# EUROPÄISCHE PATENTSCHRIFT

(11) **EP 1 574 187 B1**
(45) Veröffentlichungstag und Bekanntmachung des Hinweises auf die Patenterteilung: **06.01.2010**
(21) Anmeldenummer: 05001852.2
(22) Anmeldetag: 28.01.2005
(51) Int. Cl.: A61F 2/52

(54) **Brustprothese**
Breast prothesis
Prothèse mammaire

(30) Priorität: 03.03.2004 DE 202004003278 U
(43) Veröffentlichungstag der Anmeldung: 14.09.2005
(73) Patentinhaber: Thämert Orthopädische Hilfsmittel GmbH & Co. KG, 30938 Burgwedel/Grossburgwedel (DE)
(72) Erfinder: Schneider-Nieskens, Reinhold, 29223 Celle (DE)
(74) Vertreter: Siekmann, Gunnar

(56) Entgegenhaltungen:
- EP-A- 0 005 275
- EP-A- 0 791 345
- US-A- 4 172 298
- US-A- 5 480 429
- US-A- 5 855 606
- US-A1- 2003 195 623
- US-B1- 6 520 989

## Beschreibung

Die Erfindung betrifft eine Brustprothese, insbesondere aus Silikon mit einem äußeren schalenförmig ausgebildetem Bereich und einem inneren schalenförmig ausgebildeten Bereich, wobei der innere Bereich und der äußere Bereich derart ausgebildet sind, daß sie unterschiedliche Eigenschaften aufweisen.

Eine Brustprothese der eingangs genannten Art ist beispielsweise aus der EP 0 320 590 B2 bekannt. Bei der bekannten Brustprothese ist es vorgesehen, die Brustprothese in einem äußeren Körper mit einer Härte auszubilden, die der weichelastischen Nachgiebigkeit des natürlichen Brustgewebes angeglichen ist und einen inneren Körper oder inneren Bereich mit einer weicheren Konsistenz auszubilden, so daß sie sich dem Narbenbereich anpassen kann. Weitere Zwei-Kammer-Brustprothesen sind aus der EP-A-0 791 345 und der EP-A-0 005 275 bekannt. Ein-Kammer-Brustprothesen sind aus der US-B1-6,520,989 und US-A-4,172,298 bekannt.

Der Erfindung liegt die Aufgabe zugrunde, eine Brustprothese der eingangs genannten Art zu schaffen, die besonders gute Formeigenschaften und Verformungseigenschaften aufweist und auch besonders leicht ausgebildet werden kann.

Die Lösung dieser Aufgabe erfolgt mit einer Brustprothese mit den Merkmalen des Schutzanspruchs 1. Vorteilhafte Weiterbildungen der Erfindung sind in den Unteransprüchen beschrieben.

Bei einer Brustprothese, insbesondere aus Silikon, mit einem inneren schalenförmigen und einem äußeren schalenförmigen Bereich, wobei der innere Bereich und der äußere Bereich derart ausgebildet sind, daß sie unterschiedliche Eigenschaften aufweisen, ist es erfindungswesentlich, daß der innerhalb des äußeren Bereichs, also der auf der Innenseite der Brustprothese angeordnete innere Bereich, eine profilierte Ringstruktur aus konzentrisch umlaufenden Ringen oder Wülsten und mindestens einen zwischen den Wülsten angeordneten ringförmigen Bereich mit dünnerer Materialstärke aufweist. Durch die Ausbildung einer derartigen profilierten Struktur besteht die Möglichkeit, eine zusätzliche mechanische Stabilität zu erzeugen. Dadurch ist es möglich, auch gezielt leichte Materialien zu verwenden. Außerdem kann durch eine besondere Profilierung auch zusätzlich ein besonders wünschenswerter Effekt bei der Formgebung bzw. Verformung erreicht werden.

Erfindungsgemäß ist die profilierte Struktur als Ringstruktur, insbesondere als konzentrische Ringstruktur ausgebildet. Die Ringe sind dabei bevorzugt konzentrisch um die Mamille angeordnet. Es wird auch eine spezielle Rippen- oder Wulstgeometrie verwendet werden, die der Brustprothese, ähnlich einer Verrippung bei Leichtbauteilen, eine zusätzliche Stabilität geben. In einer bevorzugten Ausführungsform weist der profilierte innere Bereich mindestens zwei, insbesondere drei, umlaufende Ringe auf. Durch diese Ringe, die auch wulstartig ausgebildet sein können, wird erreicht, daß diese sich bei Druck auf die Prothesenspitze balgenförmig ineinanderschieben. Bevorzugt sind diese Ringe unterbrochen. In einer besonders bevorzugten Ausführungsform erfolgt die Unterbrechung der Ringe horizontal durch die Mamille verlaufend. Weiterhin ist bevorzugt auch eine vertikal verlaufende Unterbrechung der Ringe vorgesehen, die insbesondere vertikal durch die Mamille verläuft und insbesondere ausschließlich den mittleren und den inneren Ring unterbricht. Ergänzend ist bevorzugt zusätzlich oder alternativ eine Unterbrechung des mittleren Ringes eine oder mehrere Unterbrechungen, bevorzugt in der unteren Hälfte der Brustprothese vorgesehen, wobei diese bevorzugt unter einem Winkel von jeweils 45° zur Senkrechten ausgerichtet sind. Dadurch wird erreicht, daß sich die Prothese, ähnlich der natürlichen Brust, in das BH-Körbchen hineinlegt. Bei Druck auf die Prothesenspitze bewirken die konzentrischen Verrippungen oder Auswulstungen ein balgenförmiges Legen oder Schieben des Prothesenmaterials. Dadurch kann die Brustprothese sich bei auf dem Rücken liegenden Frauen abflachen. Ebenfalls kann sie sich bei Körperkontakt mit anderen Personen abflachen. In einer anderen bevorzugten Weiterbildung der Erfindung sind in der Brustprothese Soll-Faltungsausnehmungen vorgesehen. Diese sind bevorzugt zwischen den Wülsten angeordnet und ringförmig ausgebildet.

In einer anderen bevorzugten Weiterentwicklung der Erfindung ist der innere schalenförmige Bereich aus einem Leichtsilikon ausgebildet. Das Leichtsilikon wird bevorzugt aus einem Silikongel unter Einmischung von sehr kleinen Hohlkugeln hergestellt, so daß dieses Leichtsilikon insgesamt eine gegenüber normalem Silikongel sehr viel geringere Dichte aufweist. Dadurch wird insgesamt eine Gewichtsreduzierung erreicht. Durch die erfindungsgemäße Ausgestaltung der Brustprothese wird trotzdem eine ausreichende Stabilität erzielt. Bevorzugt ist der innere Bereich mit im Vergleich zu üblichen Leichtsilikonprothesen besonders fest eingestelltem Silikon ausgebildet. Insbesondere ist der innere Bereich so eingestellt, daß er einen Penetrationswert von ca. 180 hat. Demgegenüber ist die äußere Schicht bevorzugt aus Normalsilikon bzw. einem Normal-Silikongel gebildet. Dieses ist entgegen üblicher Einstellungen bevorzugt relativ weich, insbesondere mit einem Penetrationswert von ca. 260, eingestellt. Der äußere Bereich ist daher bevorzugt weicher als der innere Bereich eingestellt.

In einer anderen bevorzugten Ausgestaltung der Erfindung ist der äußere schalenförmige Bereich oberhalb der Mamille etwas dicker ausgebildet als unterhalb der Mamille. Bevorzugt beträgt das Verhältnis der Dicke des äußeren Bereichs oberhalb der Mamille zur Dicke unterhalb der Mamille etwa 1,5. In einer anderen Weiterentwicklung der Erfindung wird die Brustprothese als Dreikammerprothese ausgebildet, wobei bevorzugt zwischen dem äußeren Bereich und dem inneren Bereich dieser dritte schalenförmige Bereich vorgesehen ist. In einer weiteren bevorzugten Ausgestaltung der Erfindung weist die Brustprothese im unteren Bereich eine Ausnehmung auf. Durch diese kann bei Druck auf die Prothese das eingeschlossene Luftpolster gezielt und ohne Geräusche entweichen.

Nachfolgend wird die Erfindung anhand eines in der Zeichnung dargestellten bevorzugten Ausführungsbeispiels weiter erläutert. Im Einzelnen zeigen die schematischen Darstellungen in:
Fig. 1: Einen Vertikalschnitt durch eine erfindungsgemäße Brustprothese; und
Fig. 2: eine rückwertige Ansicht der erfindungsgemäßen Brustprothese.

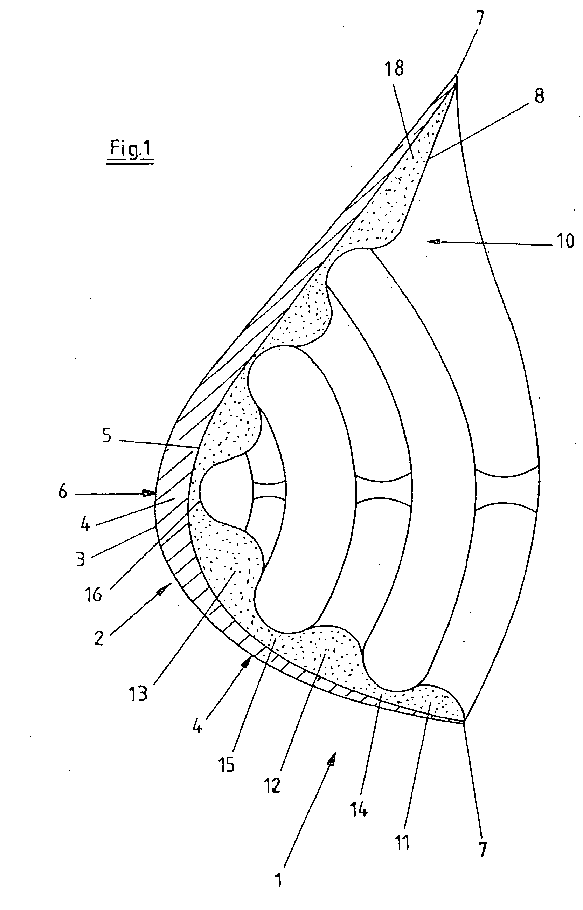

In Fig. 1 ist eine vertikale Schnittansicht der Brustprothese 1 mit dem schalenförmigen äußeren Bereich 2 und dem schalenförmigen inneren Bereich 10 dargestellt. Der äußere Bereich 1 stellt die außenliegende und von außen sichtbare Seite der Brustprothese 1 dar. Eine Silikongelschicht 4 ist in einem Folienbeutel aufgenommen, der von einer äußeren Folie 3 und einer inneren Folie 5 gebildet wird und zwischen sich die Silikongelschicht 4 aufnimmt. Diese besteht aus einem sehr weich eingestellten Silikongel mit einem Penetrationswert von ca. 260. Die Silikongelschicht des äußeren Bereichs 2 ist dabei nicht konstant ausgebildet, sondern oberhalb der Mamille 6 dicker ausgebildet als unterhalb der Mamille 6. Das Dickenverhältnis "Dicke oberhalb Mamille zu Dicke unterhalb Mamille" beträgt dabei etwa 1,5. In einem umlaufenden Endbereich 7 liegen die Folien 3 und 5 aufeinander und sind miteinander verschweißt. Weiterhin ist in diesem Endbereich 7 auch eine dritte Folie 8 mit eingeschweißt, die den unteren Abschluß des inneren schalenförmigen Bereichs 10 bildet, der zwischen der Folie 5 und der Folie 8 aufgenommen ist. Dieser der Innenseite zugewandte schalenförmige Bereich 10 ist profiliert ausgebildet. Dies wird durch Verwendung eines entsprechend profilierten Formwerkzeuges erreicht, das beim ausvulkalisieren des Silikons von innen in die Brustprothese eingeführt ist. Der innere schalenförmige Bereich 10 ist dabei mit konzentrisch umlaufenden Ringen oder Wülsten 11, 12 und 13 ausgebildet. Zwischen den Wülsten sind ringförmige Bereiche dünnere Materialstärke 14 und 15 vorgesehen, die auch als Soll-Faltungsausnehmungen bezeichnet werden können. Der Formkörper, mit dem der innere Bereich in diese profilierte Struktur gezwungen wird, weist bevorzugt die gleichen Radien im Bereich der Wülste 11, 12 und 13 wie auch im Bereich der dazwischenliegenden Ausnehmungen 14 und 15 auf. Die Wülste und Ausnehmungen entsprechen sich daher in ihren Abmessungen. Im Bereich der Mamille 6 ist der innere schalenförmige Bereich 10 mit einem sehr dünnen zentralen Bereich 16 ausgebildet. Um diesem Bereich 16 herum verläuft konzentrisch der Wulst 13, daran anschließend die umlaufende Vertiefung 15, dann wiederum ein Wulst 12, eine Vertiefung oder Soll-Faltungsausnehmung 14 und ein weiterer Wulst oder Ring 11. Der äußerste Ring oder Wulst 11 ist besonders breit ausgebildet und zum äußeren Rand 17 hin abgeflacht ausgebildet, so daß sich dort eine besonders gute Kontaktierung der Brustprothese 1 mit der Haut der Trägerin ergibt. In einem oberen Abschnitt 18 ist der äußere Wulst 11, insbesondere der Rand 17, als langer Auslauf in der Spitze ausgebildet, so daß dort eine besonders gute Kontaktierung erfolgen kann. Horizontal durch den Bereich 16, also den Bereich der Mamille, ist in dem inneren, körperzugewandten schalenförmigen Bereich 10 eine Ausnehmung 19 in den Ringen oder Wülsten 11, 12 und 13 vorgesehen.

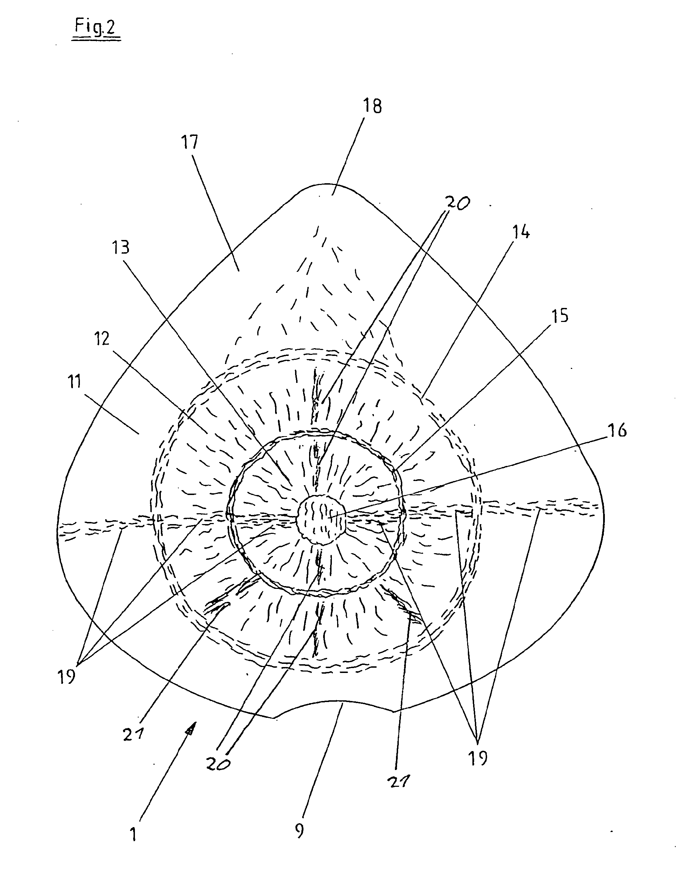

In Fig. 2 ist eine Ansicht der Brustprothese 1 von der Rückseite, also der körperzugewandten Seite, dargestellt, bei der insbesondere der innere schalenförmige Körper 10 der Brustprothese 1 zu erkennen ist. Darin sind vor allem der besonders dünne zentrale Bereich 16 der Mamille sowie die weiteren umlaufenden Ausnehmungen 14 und 15 sowie die Ringe 11, 12 und 13 zu erkennen. Ebenfalls deutlich zu erkennen, ist die Unterbrechung 19 der Ringe 11, 12 und 13, die sich horizontal über die gesamte Brustprothese erstreckt. Weiterhin sind bevorzugt Unterbrechungen 20 der Ringe vorgesehen, insbesondere der Ringe 12 und 13, also des mittleren und des inneren Ringes, die vertikal durch die Brustprothese und auch durch die Mamille verlaufen. Weiterhin sind bevorzugt Unterbrechungen 21 vorgesehen. Diese sind bevorzugt ausschließlich im mittleren Ring 12 angeordnet und jeweils unter 45° zur Senkrechten, also insbesondere auch zu den Unterbrechungen 20 ausgerichtet und bevorzugt nur in der unteren Hälfte der Brustprothese vorgesehen. Im unteren Endbereich der Brustprothese 1 ist eine Ausnehmung 9 vorgesehen. Diese Ausnehmung 9 ist halb rund ausgebildet und unterbricht den am Körper anliegenden Rand 17. Durch die Ausnehmung kann das eingeschlossene Luftpolster bei Druck gezielt und ohne Geräusche entweichen und nach dem Schwimmen kann auch das eingeschlossene Wasser ablaufen.

## Patentansprüche

1. Brustprothese zum äußerlichen Tragen, insbesondere aus Silikon, mit einem äußeren schalenförmig ausgebildeten Bereich (2) und einem inneren schalenförmig ausgebildeten Bereich (10), wobei der innere Bereich (2) und der äußere Bereich (10) derart ausgebildet sind, daß sie unterschiedliche Eigenschaften aufweisen,
**dadurch gekennzeichnet,**
**daß** der innerhalb des äußeren Bereichs (2), also der auf der Innenseite der Brustprothese angeordnete innere Bereich (10), eine profilierte Ringstruktur aus konzentrisch umlaufenden Ringen oder Wülsten (11) und mindestens einen zwischen den Wülsten angeordneten ringförmigen Bereich mit dünnerer Materialstärke (14, 15) aufweist.

2. Brustprothese nach Anspruch 1, **dadurch gekennzeichnet, daß** die Ringstruktur bzw. die Ringe (11, 12, 13) Unterbrechungen (19, 20, 21) aufweist.

3. Brustprothese nach einem der Ansprüche 1 oder 2, **dadurch gekennzeichnet, daß** der innere Bereich Soll-Faltungsausnehmungen (14, 15) aufweist.

4. Brustprothese nach einem der vorhergehenden Ansprüche, **dadurch gekennzeichnet, daß** der innere Bereich (10) aus Leichtsilikon ausgebildet ist.

5. Brustprothese nach einem der vorhergehenden Ansprüche, **dadurch gekennzeichnet, daß** der innere Bereich (10) aus einem vergleichsweise fest eingestelltem Silikon, insbesondere mit einem Penetrationswert von ca. 180 ausgebildet ist.

6. Brustprothese nach einem der vorhergehenden Ansprüche, **dadurch gekennzeichnet, daß** der äußere Bereich (2) aus Normalsilikon gebildet ist.

7. Brustprothese nach einem der vorhergehenden Ansprüche, **dadurch gekennzeichnet, daß** der äußere Bereich (2) aus vergleichsweise weich eingestelltem Silikon, insbesondere mit einem Penetrationswert von ca. 260 ausgebildet ist.

8. Brustprothese nach einem der vorhergehenden Ansprüche, **dadurch gekennzeichnet, daß** der äußere Bereich (2) weicher ausgebildet ist als der innere Bereich (10).

9. Brustprothese nach einem der vorhergehenden Ansprüche, **dadurch gekennzeichnet, daß** der äußere Bereich (2) oberhalb der Mamille (6) etwas dicker ausgebildet ist als unterhalb der Mamille (6).

10. Brustprothese nach einem der vorhergehenden Ansprüche, **dadurch gekennzeichnet, daß** die Brustprothese (1) im unteren Endbereich eine Ausnehmung (9) aufweist.

## Claims

1. A breast prosthesis for being worn externally, made of silicone in particular, having an outer area (2) designed in the form of a shell and an inner area (10) designed in the form of a shell, such that the inner area (2) and the outer area (10) are designed so that they have different properties,
**characterized in that**
the inner area (10) arranged inside the outer area (2), i.e., on the inside of the breast prosthesis, has a profiled ring structure of concentric rings or ridges (11) and at least one ring-shaped area with a thinner material thickness (14, 15) arranged between the ridges.

2. The breast prosthesis according to claim 1,
**characterized in that** the ring structure and/or the rings (11, 12, 13) have interruptions (19, 20, 21).

3. The breast prosthesis according to one of claims 1 or 2, **characterized in that** the inner area has predetermined fold recesses (14, 15).

4. The breast prosthesis according to any one of the preceding claims, **characterized in that** the inner area (10) is formed from lightweight silicone.

5. The breast prosthesis according to any one of the preceding claims, **characterized in that** the inner area (10) is formed from a silicone, which is formulated to be comparatively solid, in particular having a penetration value of approximately 180.

6. The breast prosthesis according to any one of the preceding claims, **characterized in that** the outer area (2) is formed from normal silicone.

7. The breast prosthesis according to any one of the preceding claims, **characterized in that** the outer area (2) is formed from comparatively soft silicone, in particular having a penetration value of approximately 260.

8. The breast prosthesis according to any one of the preceding claims, **characterized in that** the outer area (2) is designed to be softer than the inner area (10).

9. The breast prosthesis according to any one of the preceding claims, **characterized in that** the outer area (2) above the nipple (6) is designed to be somewhat thicker than that below the nipple (6).

10. The breast prosthesis according to any one of the preceding claims, **characterized in that** the breast prosthesis (1) has a recess (9) in the lower end area.

## Revendications

1. Prothèse mammaire se portant à l'extérieur, notamment en silicone, comportant une partie extérieure (2) réalisée en forme de coque et une partie intérieure (10) réalisée en forme de coque, la partie intérieure (2) et la partie extérieure (10) étant réalisées de manière à présenter différentes propriétés,
**caractérisée en ce que**
la partie intérieure (10) disposée à l'intérieur de la partie extérieure (2), donc sur la face interne de la prothèse mammaire, présente une structure annulaire profilée faite d'anneaux ou bourrelets (11) périphériques concentriques et au moins une partie de forme annulaire disposée entre les bourrelets et ayant une épaisseur plus mince de matériau (14, 15).

2. Prothèse mammaire selon la revendication 1, **caractérisée en ce que** la structure annulaire ou les anneaux (11, 12, 13) présente(nt) des brèches (19, 20, 21).

3. Prothèse mammaire selon une des revendications 1 ou 2, **caractérisée en ce que** la partie intérieure présente des évidements de pliage théorique (14, 15).

4. Prothèse mammaire selon une des revendications précédentes, **caractérisée en ce que** la partie intérieure (10) est réalisée en silicone léger.

5. Prothèse mammaire selon une des revendications précédentes, **caractérisée en ce que** la partie intérieure (10) est réalisée en silicone de type relativement solide, notamment avec une valeur de pénétration d'environ 180.

6. Prothèse mammaire selon une des revendications précédentes, **caractérisée en ce que** la partie extérieure (2) est composée de silicone normal.

7. Prothèse mammaire selon une des revendications précédentes, **caractérisée en ce que** la partie extérieure (2) est composée de silicone de type relativement mou, ayant notamment une valeur de pénétration d'environ 260.

8. Prothèse mammaire selon une des revendications précédentes, **caractérisée en ce que** la partie extérieure (2) a une conformation plus molle que la partie intérieure (10).

9. Prothèse mammaire selon une des revendications précédentes, **caractérisée en ce que** la partie extérieure (2) a une conformation un peu plus épaisse au dessus du sein (6) qu'en dessous du sein (6).

10. Prothèse mammaire selon une des revendications précédentes, **caractérisée en ce que** la prothèse mammaire (1) présente un évidement (9) dans sa partie inférieure étroite.
